# EUROPEAN PATENT APPLICATION

(11) **EP 2 292 629 A1**
(43) Date of publication of application: **09.03.2011**
(21) Application number: 10009175.0
(22) Date of filing: 03.09.2010
(51) Int. Cl.: C07G 5/00, C07D 471/18, A61K 31/439, A61P 43/00

(54) **Method of isolating cytisine from biomaterial**

(30) Priority: 07.09.2009 DE 102009040380
(71) Applicant: Kallimopoulos, Thomas, Dr., 67059 Ludwgishafen (DE)
(72) Inventor: Kallimopoulos, Thomas, Dr., 67059 Ludwgishafen (DE)
(74) Representative: Flaccus, Rolf-Dieter

(57) **Abstract**

The present invention concerns a method for extracting cytisine from biomaterial wherein the biomaterial is contacted with an alkaline aqueous solution, but in the absence of any other co-solvent such as an organic phase. Cytisine is recovered from the alkaline aqueous extract by extraction of the extract with rapeseed oil which in turn is extracted with an acidic aqueous phase.

## Description

The present invention concerns methods for isolating cytisine from biomaterial.

Cytisine (CAS-No. 485-35-8) is a pyridine-like alkaloid and known to be a potent nicotinic acetylcholine receptor agonist. Pharmacologically, cytisine exhibits similar effects as nicotine.

Cytisine is used as compound in pharmaceutical preparations for treating addictions to various drugs, in particular for treating addiction to nicotine and addiction to alcohol. For example, natural cytisine is commercially available under the trade name Tabex in eastern European Countries. Varenicline, a derivative of cytisine, is offered by Pfizer as Chantrix^{®} to treat addictions to tobacco products.

It is known that various plant species contain cytisine. For example, cytisine was found in species from several genera of the Faboideae subfamily, including *Laburnum, Anagyris, Thermopsis, Cytisus, Genista* and *Sophora.* Cytisine is also present in *Gymnocladus,* a member of the Caesalpinioideae subfamily.

Plants containing cytisine, including the Common Broom (*Cytisus scoparius*) and Mescalbean (Calia spec.) have been used recreationally. Positive effects are reported to include a mild intoxication and heightened awareness of color. However this practice is not recommended since negative side-effects can include nausea, vomiting, convulsions, heart pain, headache and in larger doses even death via respiratory failure. Also mamane (*Sophora chrysophylla*) can contain amounts of cytisine that are lethal to most animals, and seeds of *Laburnum anagyroides* can contain cytisine in an abundance of 0.1 to 2%.

The extraction of cytisine has been described in the literature as somehow tedious, requiring either large quantities of solvents, long extraction times and/or the use of special equipment for extracting sufficient amounts of cytisine.

The extraction of cytisine from seeds of Laburnum has been reported in the literature more than a century ago (Partheil, A., Ber. D. Ch. G. 23, 3201 (1890)).

Later, Klein (Klein, C., Handbuch der Pflanzenanalyse, Vienna (1933), Ed. J. Springer) has extracted cytisine by submitting powdered seeds in alkali to form a paste. Then a crude extract was obtained by extracting the paste with chloroform in a Soxhlet apparatus for several hours.

Danil'chuk (Danil'chuk, D.N., Uzb. Khim. Zh. 2, 29-31 (1984)) used liquid ammonia to extract cytisine from *Thermopsis* and *Sophora* species.

More recently, Wink (Wink, E., Pharmazie 51, 768 (1996)) had to use a mixture of CH₂Cl₂, methanol and NH₄OH (25%) to extract cytisine from homogenized plant material.

Most recently, a method of extracting cytisine has been described in "Organic Synthesis", wherein almost the same method utilized as the one used by Wink, and wherein the entire process took more than 69 hours (Organic Synthesis 83, 141 (2006)).

In addition to aforementioned scientific literature, laid open publication DE 34 29 543 A1 discloses a method for isolating alkaloids from biomaterial, wherein cytisine was obtained from a homogenized mixture of ground seeds with 0.1 N HCl in that the alkaloids were bound to and eluted from an ion exchange resin.

In view of the interest of the scientific community and pharmaceutical industry in cytisine, there is a demand for a method of isolating cytisine in larger quantities and without the drawbacks of those methods that were described in the literature thus far.

The problem is solved by the method of the present invention, wherein cytisine is isolated from biomaterial, preferably plant biomaterial, using a combination of extraction steps. The method of the present invention is simple and expansible to large amounts of biomaterial without necessity to set-up special technologies.

In the method of the present invention, on one hand the solubility of cytisine in water (439 g/l) is exploited and in a vegetable oil on the other hand.

In the method of the present invention, cytisine-containing biomaterial is comminuted if necessary, and mixed with alkalized water to obtain an aqueous alkaline extract containing cytisine.

Although some biomaterial such as unicellular organisms does not require comminution prior to being extracted, it is preferred that the biomaterial, in particular plant biomaterial, is comminuted prior to the extraction. Comminuting means that the biomaterial is mashed, cut in small pieces, broken, coarsed, milled, ground, pulverised or comminuted by any other suitable means.

"Alkalized water" refers to an aqueous alkaline solution. In the process of the present invention, the initial extraction of biomaterial is performed by utilizing an alkaline aqueous solution. The alkaline aqueous phase can be an aqueous solution of ammonia, also known as ammonium hydroxide (NH₄OH). In another embodiment, the alkaline aqueous solution is an aqueous solution of an alkali metal carbonate, preferably an aqueous solution of sodium carbonate (Na₂CO₃) or potassium carbonate (K₂CO₃). In another embodiment, the alkaline aqueous phase is an aqueous solution of an alkali metal hydrogen carbonate, preferably an aqueous solution of sodium hydrogen carbonate (NaHCO₃) or potassium hydrogen carbonate (KHCO₃). In still another embodiment, the alkaline aqueous solution is an aqueous solution of an alkali metal hydroxide, preferable an aqueous solution of sodium hydroxide (NaOH) or potassium hydroxide (KOH). Preferred aqueous alkaline solutions are aqueous solutions of either NH₄OH or Na₂CO₃. More preferably, the aqueous alkaline solutions contain 5% (w/w) of NH₄OH or Na₂CO₃.

No other co-solvent than said alkaline aqueous solution is employed in the initial extraction of the biomaterial so to avoid formation of an emulsion. This avoidance of mixing different solvents is particularly advantageous in industrial practice for safety and economical reasons.

The aqueous alkaline extract containing cytisine is then extracted with a vegetable oil to transfer the alkaloid from the aqueous phase to the oil phase, because the vegetable oil extracts cytisine but only very small amounts of n-methyl cytisine and other alkaloids. The preferred vegetable oil for extracting cytisine from the alkaline aqueous extract of cytisine-containing biomaterial is rapeseed oil.

However, other vegetable oils may also employed as organic phase for extracting cytisine from the alkaline aqueous extract of the biomaterial.

The term "vegetable oil" refers to any material from a plant that is liquid at room temperature (approx. 23°C) and composed of triglycerols, free fatty acids, monoglycerols and diglycerols.

To be a suitable solvent in the extraction process of the present invention, the vegetable oil does not require any chemical modification. Thus straight vegetable oils are preferred vegetable oils in the extraction process of the present invention.

Examples of other vegetable oils than rapeseed oil that are suitable to be used as solvent in the extraction of cytisine from the alkaline aqueous extract of biomaterial are sunflower oil, linseed oil, grape seed oil, peanut oil, castor oil, pumpkin seed oil, soy bean oil, safflower oil, cotton seed oil, coconut oil, corn oil, castor oil, palm oil, hempseed oil, rice bran oil, tung oil, jojoba oil and olive oil.

In general, any vegetable oil may be used, regardless of its origin or grade. Although industrial grade vegetable oils may be employed, it is preferred that the vegetable oil is of food grade, veterinary grade or cosmetic grade. The most preferred vegetable oils for the extraction of alkaloids from plant material are edible vegetable oil.

In the next process step of the extraction process of the present invention, the rapeseed oil/vegetable oil containing cytisine is treated with an acid to form the corresponding cytisine salt in the aqueous phase. It is preferred to use sulfuric acid, i. e. an aqueous solution of H₂SO₄ (most preferably 3% (w/w) in water), to form cytisine sulfate.

Any usual inorganic acid is equivalent to H₂SO₄ such that for instance aqueous solutions of HCl or HNO₃ may be employed instead of the aqueous solution of H₂SO₄.

Subsequently, the cytisine base is recovered from the cytisine salt in the aqueous phase by alkaline extraction of said aqueous phase using CH₂Cl₂ and a small amount of methanol to remove the remaining water. Therefore, the acidic aqueous phase is alkalised, preferably with aqueous ammonia. The preferred pH value of the alkalised aqueous phase is about pH 11. Then the aqueous phase is extracted with an organic solvent that is immiscible with the aqueous phase. The preferred organic solvent that is immiscible with the aqueous phase is CH₂Cl₂. The organic phase is recovered, dried, and the organic solvent is evaporated to obtain a dry residue which contains the alkaloid(s).

This latter process step can be supplanted by azeotropic removal of water from the dichloromethane phase without using methanol.

In a preferred embodiment of the process of the present invention, said process comprises an intermediate treatment of the aqueous cytisine salt solution with cyclohexane. The cytisine salt is insoluble in cyclohexane, and cyclohexane treatment will remove undesired residues of the oil phase from the aqueous, cytisine-containing phase such that purity of the cytisine extract is improved.

The extraction process of the present invention is robust, faster than known extraction methods for cytisine, does not require to set-up special technology, and can be scaled-up for large scale isolation of cytisine from biomass.

The extraction process of the present invention is advantageous in that the vegetable oil extracts cytisine but only traces of n-methyl cytisine and other alkaloids from an alkaline aqueous extract of cytisine-containing biomaterial. Other organic solvents such as dichloromethane, chloroform, toluene, ethanol or methanol are not that selective.

Moreover, the use of organic solvents such as dichloromethane or toluene form a thick emulsion with the plant material and the ammonia solution which is problematic with respect to further processing. This is not the case with employing a vegetable oil when the extraction of the biomaterial is performed first with an alkaline aqueous solution, removal of the biomaterial from the alkaline aqueous solution by filtration, and subsequent treatment of the filtered alkaline aqueous solution with a vegetable oil, preferably rapeseed oil. The scope of the present invention also comprises cytisine that was isolated from biomass, in particular from plant biomass, by a method of the present invention.

The present invention further extends to the use of the cytisine that was/is isolated by the method of the present invention for manufacturing a medicament, preferably for a medicament for pharmacological treatment of addictions, in particular for pharmacological treatment of addictions to alcohol and/or tobacco products/nicotine.

### Example

50 grams of powdered seeds from *Laburnum anagyroides,* 100 ml of NH₄OH (25%) and 400 ml water were introduced into an extractor and stirred at room temperature (approx. 23°C) for 20-22 hours. Thereafter, the resulting brownish suspension was filtered under pressure using a hydrapress.

The filtrate was then extracted with 250 ml rapeseed oil (trade name "Bonita" from a superstore). The oil phase was treated twice with 150 ml aqueous H₂SO₄ (3%) having a pH of 2. After clear decanting over a period of 2 hours, the acidic aqueous phase was poured into a mixture consisting of 150 ml CH₂Cl₂ and 20 ml NH₄OH (25%) and extracted at pH 10.

The aqueous phase was removed and 10 ml methanol was added to the lower organic phase to remove the remaining water from the organic phase. Water separated rapidly and clearly from the dichloromethane phase.

After evaporation of the solvent of the dichloromethane phase, i.e. the dichloromethane, 0.05 gram of a white residue was obtained which partially solidified on standing. The purity of cytisine was 28%.

The purity of isolated cytisine was increased to 55% in additional attempts in that the cytisine sulfate solution has been treated with cyclohexane before the cytisine base was generated.

## Claims

1. A method for isolating cytisine from biomaterial, the method comprising the steps of:
- contacting the biomaterial with an alkaline aqueous solution;
- extracting the alkaline aqueous solution with a vegetable oil;
- extracting the vegetable oil with an acidic aqueous phase,
- recover cytisine from the acidic aqueous phase by alkaline extraction using dichloromethane;
- removing water from the dichloromethane phase; and
- evaporating the solvent.

2. The method according to claim 1, **characterized in that** the biomaterial is a plant biomaterial.

3. The method according to claim 1 or 2, **characterized in that** the biomaterial is comminuted prior to contacting said biomaterial with an alkaline aqueous solution.

4. The method according to any one of the preceding claims, **characterized in that** the alkaline aqueous solution is an aqueous solution of an alkali metal carbonate, an aqueous solution of an alkali metal hydrogen carbonate, an aqueous solution of an alkali metal hydroxide or an aqueous solution of ammonium hydroxide.

5. The method according to any one of the preceding claims, **characterised in that** the alkaline aqueous solution is selected from the group consisting of aqueous solutions of sodium carbonate, potassium carbonate, sodium hydrogen carbonate, potassium hydrogen carbonate, sodium hydroxide and potassium hydroxide.

6. The method according to any one of the preceding claims, **characterized in that** the vegetable oil is selected from the group consisting of rapeseed oil, sunflower oil, linseed oil, grape seed oil, peanut oil, castor oil, pumpkin seed oil, soy bean oil, safflower oil, cotton seed oil, coconut oil, corn oil, castor oil, palm oil, hempseed oil, rice bran oil, tung oil, jojoba oil and olive oil, wherein rapeseed oil is particularly preferred.

7. The method according to any one of the preceding claims, **characterized in that** the acidic aqueous phase is an acidic aqueous solution, preferably an aqueous solution of H₂SO₄.

8. The method according to any one of the preceding claims, **characterized in that** the cytisine is recovered from the acidic aqueous phase by alkaline extraction **in that** the acidic aqueous phase is treated with a mixture consisting of dichloromethane and ammonia.

9. The method according to any one of the preceding claims, **characterized in that** the acidic aqueous phase is extracted with cyclohexane after its separation from the vegetable oil and prior to its alkaline extraction using dichloromethane.

10. Cytisine, extracted from biomaterial by a method according to any one of the preceding claims.

11. Use of cytisine that was extracted from biomaterial by a method according to any one of claims 1 to 10 for manufacturing of a medicament, preferably for manufacturing a medicament for treating addictions and more preferably for manufacturing a medicament for treating addictions to alcohol and/or tobacco products.
